Europäisches Patentamt

European Patent Office

Office européen des brevets

(11) Publication number: **0 092 249**
**B1**

(12) **EUROPEAN PATENT SPECIFICATION**

(45) Date of publication of patent specification: **26.10.88**

(21) Application number: **83103858.3**

(22) Date of filing: **20.04.83**

(51) Int. Cl.⁴: **A 61 K 39/42**, C 12 P 21/00,
G 01 N 33/577,
G 01 N 33/576

(54) **Non-A, non-B hepatitis-associated monoclonal antibody and diagnostic reagent.**

(30) Priority: **21.04.82 JP 65430/82**

(43) Date of publication of application:
**26.10.83 Bulletin 83/43**

(45) Publication of the grant of the patent:
**26.10.88 Bulletin 88/43**

(84) Designated Contracting States:
**BE CH DE FR GB IT LI NL SE**

(56) References cited:
**EP-A-0 027 657**
**EP-A-0 066 296**
**EP-A-0 068 465**
**WO-A-82/03330**

(73) Proprietor: **Eisai Co., Ltd.**
**6-10, Koishikawa 4-chome Bunkyo-ku**
**Tokyo 112 (JP)**

(72) Inventor: **Akatsuka, Toshitaka**
**2-34, 617, Kita Kasai 4-chome**
**Edogawa-ku Tokyo (JP)**
Inventor: **Tohmatsu, Junichi**
**1-5-511, Kitamachi 4-chome**
**Warabi-shi Saitama (JP)**
Inventor: **Shikata, Toshio**
**17-2, Motokitakata 1-chome**
**Ichikawa-shi Chiba (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

**Description**

This invention relates to non-A, non-B, hepatitis-associated monoclonal antibody, and a culture composition and a diagnostic reagent containing the same.

The existence of viral heptatis of types A and B is known and an antigen-antibody system associated with these types of hepatitis has also been clarified. Immunoserological diagnosis using such systems has become possible and development and introduction of vaccines have been accomplished.

In the course of research, however, the existence of viral hepatitis which belongs neither to type A hepatitis nor to type B hepatitis, has become known and the frequency of occurrence thereof has been higher than expected. Type non-A, non-B heptatitis accounts for more than 80% up to 90% of post-transfusion hepatitis and it has been reported that type non-A, non-B hepatitis also accounts for about 50% of sporadic acute hepatitis. It has been established that even among normal blood donors, a considerable number of donors carry the non-A, non-B hepatitis virus. The following literature references set forth the state of the art pertaining to non-A, non-B hepatitis:

1) Prince, A.M. et al.: Long-incubation post-transfusion hepatitis without serological evidence of exposure to hepatitis-B virus. Lancet. II: 241—246, 1974.

2) Alter, H.J. et al.: Clinical and serological analysis of transfusion-associated hepatitis. Lancet. II: 838—841, 1975.

3) Feinstone, S.M. et al.: Transfusion-associated hepatitis not due to viral hepatitis type A or B. N. Engl. J. Med. *292* 767—770, 1975.

4) Meyers, J.D. et al.: Parenterally transmitted non-A, non-B hepatitis, Ann. Intern. Med. *87*: 57—59, 1977.

5) Villarejos, V.M. et al.: Evidence for viral hepatitis other than type A or type B among persons in Costa Rica. N. Engl. J. Med. *293*: 1350—1352, 1975.

6) Dienstag, J.L. et al.: Etiology of sporadic hepatitis B surface antigen-negative hepatitis. Ann. Intern. Med. *87*: 1—6, 1977.

7) WO—A—82/03330

The term "non-A, non-B hepatitis" used herein refers to those kinds of hepatitis which are believed to occur because of infection with virus(es) other than the previously known viruses, such as, type A hepatitis virus, type B hepatitis virus, cytomegalovirus, Epstein-Barr virus and the like, which non-A, non-B hepatitis viruses participate in the disease as pathogens, and which do not exhibit a significant rise for HAV anti-body, anti-HBs, anti-HBc and/or anti-HBe. Accordingly, non-A, non-B patients can be defined as those who suffer from hepatitis resulting from infection by hepatitis viruses, but who are negative in both the type A hepatitis diagnosis and the type B hepatitis diagnosis, and are therefore excluded from having either type A hepatitis or type B hepatitis. Generally, in comparison with type B hepatitis, the average incubation period from transfusion until outbreak, for non-A, non-B hepatitis, is short and the maximal value of GPT is also low. However, non-A, non-B hepatitis has a longer duration so that the GPT abnormality, for example, becomes chronic and lasts longer. In any case, non-A, non-B hepatitis accounts for the major proportion of post-transfusion hepatitis. Liver diseases caused by the non-A, non-B hepatitis virus(es) include a variety of diseases such as liver cirrhosis, hepatocellular carcinoma, and the like. Accordingly, proper countermeasures must be devised as soon as possible.

Under these circumstances, some of the inventors of the present invention attempted to isolate, as a separate entity, a non-A, non-B hepatitis-associated antigen which is general as well as specific to non-A, non-B hepatitis, and succeeded in obtaining the contemplated substance by isolation and purification from non-A, non-B hepatitis autopy liver. The result of this attempt was filed for patent as Japanese Patent Application No. 83736/1981 & EP—A—66296. They further attempted to prepare a non-A, non-B hepatitis-associated antibody by immunological techniques using this substance and the fruit of this attempt was filed for patent as Japanese Patent Application No. 97425/1981 & EP—A—68465.

The inventions disclose in these patent applications can be deemed extremely significant in that they make it possible for the first time to directly diagnoze non-A, non-B hepatitis. However, when the production method of these inventions is practised, the amount of the substance obtained thereby is extremely small so that difficult problems are yet to be solved for putting the substance into practical application. Especially because large quantities of a non-A, non-B hepatitis-associated antibody is expected to be consumed not only for the diagnosis but also for the remedy of non-A, non-B hepatitis, it is necessary to obtain large quantities of the antibody by culture, for example. Since the non-A, non-B hepatitis-associated anti-body of the prior inventions is immunologically prepared using the antigen molecules having a plurality of antigenic determinants, it is given as a mixture of antibodies corresponding to these determinants. Accordingly, there is a likelihood that the mixture reacts not only with the non-A, non-B hepatitis-associated antigenic determinants but also with other antigenic determinants. To further improve the usefulness as the diagnostic reagent, therefore, it is necessary that the antibody be prepared as one that specifically couples with one of the antigenic determinants of the non-A, non-B hepatitis-associated antigen molecules.

With the background described above, the inventors of the present invention attempted to obtain a non-A, non-B hepatitis-associated monoclonal antibody applying the cell fusion techniques disclosed by Köhler and Milstein in "Nature", *256*, 495—497 (1975). As a result, we succeeded in obtaining a novel hybridoma and its culture composition which continuously produces the monoclonal antibody. The cell fusion technique allows the continuous production of a more homogeneous or so-called "monoclonal" antibody through the culture of fused cells; the general concept of its production method is already well known in the art. However, the resulting monoclonal antibody of the present invention is a novel substance and exhibits an antigen-antibody reaction specific to the non-A, non-B hepatitis-associated antigen. Accordingly, the antibody of the present invention makes it possible to specifically detect and determine the non-A, non-B hepatitis-associated antigen through the antibody sandwich method and to specifically detect and determine also the non-A, non-B hepatitis-associated antibody through the antigen inhibition method. In other words, the antibody of the present invention can provide a diagnostic reagent having a high sensitivity. The antibody of the present invention can be produced in large amount and has a high titer. In other words, the antibody having high titer can be produced in large quantities in ascites by the in vivo culture of a hybridoma producing the antibody of the present invention, especially by the intraperitoneal culture of the hybridoma.

As described above, the monoclonal antibody in accordance with the present invention is not only a novel substance but also immunologically specific to the non-A, non-B hepatitis-associated antigen. Hence, the inventive progress of the present invention resides in that it can provide a diagnostic reagent capable of determining the non-A, non-B hepatitis-associated antigen and the non-A, non-B hepatitis-associated antibody with a high level of sensitivity, that it can be produced in large amount and that it has a high titre.

Brief description of the attached drawings

Figure 1 is a drawing which corresponds to Figure 1 described in the item "Results" in Experimental Example 1 and is a schematic diagram showing the formation of the precipitin lines in accordance with the micro-Ouchterlony's test;

Figure 2 is a drawing which corresponds to Figure 2 described in the item "Results" in Experimental Example 2 and is a diagram showing the relationship between the antigen concentration and cpm in the antibody sandwich method; and

Figure 3 is a drawing which corresponds to Figure 3 described in the item "Results" in Experimental Example 3 and is a diagram showing the relationship between the serum dilution and the antigen inhibition ratio in the RIA inhibition method.

Now, the construction of the present invention will be described.

As disclosed in detail in the aforementioned Japanese Patent Application No. 83736/1981, the non-A, non-B hepatitis-associated antigen in the present invention can be obtained from the hepatitis autopsy liver of non-A, non-B hepatitis patients, as the starting material, by a suitable combination of conventional protein fractionation methods, such as density gradient centrifugation, salting out, electrophoresis, gel filtration, affinity chromatography, isoelectrofocusing, ultra-filtration, Cohn's fractionation, and so forth. In a preferred embodiment of the invention, the autopsy liver homogenate supernatant is first subjected to column chromatography using Sephacryl® S-200 and the resulting antigen positive fraction is subjected to ultrafiltration for the purpose of vacuum concentration. Then ultracentrifugation and dialysis are finally carried out. It is preferred to carry out ultracentrifugation once or several times by using successively both sucrose density gradient centrifugation and cesium chloride density gradient centrifugation. The purified matter thus obtained shows a clear precipitin line with serum of the convalescent phase from a non-A, non-B hepatitis patient by agarose gel diffusion, and it is confirmed that the precipitin line completely merges with that in the autopsy liver homogenate supernatant before purification.

The antigen has the following physicochemical properties. It has a molecular weight of at least 1,500,000 as determined by a gel filtration method. The sedimentation constant $(10^{-13})$ is 51.5S when measured with a Beckmann Model E ultracentrifuge. The buoyant density $(g/cm^3)$ in a cesium chloride or potassium bromide solution is 1.15 to 1.25 when measured with an Abbe's refractometer. The particle size (nm) ranges from 26 to 37 (31.5 on the average) under electron microscope observation. The electrophoretic mobility is in the $\alpha_2$—$\alpha_1$ globulin range when determined by immunoelectrophoresis.

The antigen has the following immunological properties.

First, the antigen undergoes a precipitation reaction in an agarose gel with the serum of a convalescent phase from non-A, non-B hepatitis patients and with that of multiply-transfused hemophilia patients. Sheep erythrocytes sensitized by the antigen exhibit specific and highly sensitive passive hemagglutination with the serum of a convalescent phase from non-A, non-B hepatitis patients. On the other hand, the antigen does not react with HAV antibody, anti-HBs, anti-HBc, anti-HBe, anti-δ, HC antibody, HB antibody, Arai antibody, F antibody, normal human liver supernatant antibody and various normal human plasma antibodies. Accordingly, this antigen is found to be a specific antigen associated with non-A, non-B hepatitis as well as general to non-A, non-B hepatitis.

An immunized antibody to this antigen can be produced by repeatedly dosing immunized rabbits with the antigen together with a suitable adjuvant such as Freund's complete adjuvant. This object can be accomplished, for example, by subcutaneously injecting BALB/C mice as the immunized animal with the antigen in an amount of 1 to 20 μg/animal for the first dose and after the passage of 90 to 120 days, injecting intravenously the animals with the antigen in an amount of 1 to 20 μg/animal without the adjuvant for the second dose. In the examples below, the antigen is dissolved in a phosphate-buffered saline (PBS) in 85 μg/ml, and 2 ml of the Freund's complete adjuvant is added to 0.2 ml of this saline. The first subcutaneous injection is made to the BALB/C mice and, after the passage of 100 days, the intraveneous injection of the same amount of the antigen solution is made.

Next, the antibody-producing cells are collected from the immunized animal. It is generally convenient to use the pulpar cells. For example, the spleen of the BALB/C mouse is enucleated after the passage of three days from the second dose and is then mulled into pieces in an RPMI-1640 medium and the resulting suspension is washed thrice by centrifugation and decanting inside the RPMI-1630 medium. The cell pellet is again suspended in the RPMI-1640 medium for the purpose of adjustment.

The myeloma cell type P3-NSI/1-Ag4-1 used for cell fusion is derived from the BALB/C mouse MOPC21, as disclosed by Köhler et al. in "Eur. J. Immunol." Vol. 6 (1976), 292—295, for example. This will be hereinafter referred to as the "myeloma cell NS-1". The cells of this kind are resistant to 8-azaguanine and since they lack enzyme hypoxanthine guanine phosphoribosyltransferase, they extinguish in a medium containing hypoxanthine-aminopterinthymidine (HAT medium).

Subculture is effected in an RPMI-1640 medium supplemented with 20 μg/ml of 8-azaguanine and 10% v/v of fetal calf serum and the medium is replaced within 4 days. Selection and cloning by the HAT medium after fusion are effected in an RPMI-1640 medium supplemented with 15% v/v fetal calf serum.

Polyethylene glycol having an average molecular weight of about 1,000 to about 6,000 is generally used as the fusing agent for cell fusion and polyethylene glycol 1500 is reported to exhibit the highest efficiency. The optimal density is preferably from 40 to 50% w/v depending upon the molecular weight of polyethylene glycol. If polyethylene glycol 1500 is used, for example, 1 ml per gram of the glycol of an RPMI-1640 not containing any fetal calf serum is dissolved therein and the solution is heated to 37°C at the time of use. Cell fusion is effected in the following manner.

First, the antibody-producing cells such as pulpar cells and myeloma NS-1 described above are washed with the RPMI-1640 not containing any fetal calf serum and are then mixed in a mixing ratio of 4:1 to 10:1 between the former and the latter in terms of the number of cells. 800 g of the mixture is centrifuged for 5 minutes to form a pellet and the medium is completely removed by decantation. The fusing agent heated to 37°C is then added dropwise with stirring at a rate of 1 ml per about 1 to $2 \times 10^8$ cells. While stirring is being continued, 10 ml of the serum-free medium is gradually added in the course of 5 to 10 minutes, for example, and 400 g is finally centrifuged for 5 minutes to remove polyethylene glycol. The residue is suspended again in 20 to 200 ml of RPMI-1640 containing 15% v/v fetal calf serum to complete the fusion.

Next, isolation from the parent cell can be effected by incubation in the HAT medium in the following manner (HAT selection). First, 0.2 ml of the suspension described above (number of cells: $10^5$ to $10^6$) is placed in each hole of a tissue culture plate having 96 holes. The half of the medium is replaced by the HAT medium every two to four days. From 14th days onward, the replacement of the medium is made at least twice by an HT medium, followed then by an RPMI medium.

In this HAT selection, the parent cells are extinguished and only the fused cells remain. Next, only the cells that produce the intended non-A, non-B hepatitis-associated antibody are screened from the residual fused cells while checking is made whether or not the intended antibody is being produced, by a suitable method such as a passive hemagglutination process (PHA process). In the examples below, the antibody producibility is judged positive if the agglutation titer is at least four times as much when measured by the PHA process.

Next, the antibody positive fused cells are fractionated and incubated by a suitable method to prepare a monoclone cell strain originating from single cells. This procedure is referred to as "cloning" and the cloning method is preferably a limiting dilution method as described below.

That is, the cell suspension is diluted to a concentration of 15 cells/ml using an RPMI-1640 medium which is supplemented with fetal calf serum and with thymocyte of the BALB/C mouse as the feeder. The suspension is placed in a tissue culture plate having 96 holes at a rate of 0.2 ml (3 cells/hole) and the antibody producibility of the incubation supernatant of the hole or holes in which cell multiplication is observed is checked in the same way as screening.

This cloning operation by limiting dilution is repeated three times in all at a rate of one cell/hole.

As will be described in the examples below, the results obtained by the procedures ranging from the finish of the fusion till the end of the cloning are as follows. Among 315 holes of the tissue culture plates having 96 holes in which incubation is first made after fusion, 150 holes show the presence of the cells in the HAT mediums. Among these 150 holes, 16 holes contain the cells having an antibody producibility. From these holes, monoclonal cell strains of 14 clones are finally obtained by the cloning operation described above.

The monoclonal antibody in the incubation supernatant obtained by in vitro incubation of these strains is precipitated by an ammonium sulfate process, and is dialyzed by PBS. By performing analysis for immunoglobulin by the micro-Ouchterlony's test (MO), the presence of $I_gG1$ or $I_gM$ was confirmed.

The positive hybridoma that is monocloned by cloning can be multiplied by in vitro or in vivo incubation in the following manner.

In vitro incubation is a method which can afford a pure monoclonal antibody not containing other immunoglobulins. This can be effected by incubating the positive hybridoma in accordance with the present invention in a suitable nutrient medium such as an RPMI-1640 which is supplemented with fetal calf serum.

In vivo incubation is a method which results in slight contamination with other immunoglobulins but can produce large quantities of monoclonal antibody. This method can be practiced in the following manner. Pristane (2,6,10,14-tetramethylpentadecane) is abdominally administered in advance to a BALB/C mouse and, after the passage of at least two days, the positive hybridoma in accordance with the present invention is abdominally administered so as to multiply and fix the antibody as the ascites tumor inside the body of the mouse. After incubation, ascitic and/or blood serums are collected and subjected to isolation-purification procedures such as salting out, chromatography and the like, to provide the monoclonal antibody of the present ivention. For example, as will be illustrated below in the examples, 0.5 ml of pristane is abdominally administered to a BALB/C mouse and, after 2 to 30 days, $2{\sim}4{\times}10^6$ positive hybridomas are abdominally administered. Two to three weeks later, ascitic serum is collected and an antibody activity specific to the non-A, non-B hepatitis-associated antigen is confirmed by the PHA and MO methods. The immunoglobulin is then fractionated by chromatography using Sephacryl® S-200 and the positive fraction is confirmed by the PHA method and is then pooled.

As will be illustrated later in the examples, the monoclonal antibody of the present invention exhibits a specific antigen-antibody reaction with the non-A, non-B hepatitis-associated antigen. This is the property that characterizes the antibody of the present invention. By utilizing this property, there can be provided a diagnostic reagent which can extremely sensitively detect and determine the non-A, non-B hepatitis-associated antigen as well as the non-A, non-B hepatitis-associated antibody, as will be also illustrated in the examples. The diagnostic reagent obtained thereby contains the monoclonal antibody of the present invention as the essential and principal component.

The diagnostic reagent of the present invention may utilize any immunological test methods using the antibody of the present invention and can be suitably prepared in accordance with the immunological test methods to be employed. When a method such as micro-Ouchterlony's test (MO), immunoelectrophoresis (IES, IEP), complement fixation (CF), immune adherence hemagglutination (IAHA) or single radial immunodiffusion (SRID) is used, the antibody of the present invention may be used as such in a suitable preparation. When, for example, the single radial immunodiffusion method is used, a suitable support is first selected from the group consisting of agar, agarose, starch, and polyacrylamide gel, and the support is heat-dissolved in a buffer. Then the antibody of the present invention is added thereto and the resulting solution is caused to flow onto a glass plate or charged into a plastic container and then cooled to effect solidification. Finally a hole for charging the serum to be tested is bored on the solidified gel plate.

When the reverse passive hemagglutination (R-PHA) method, the radioimmunoassay (RIA) method, the enzyme-linked antibody (EIA) method are used as the test method, the antibody of the present invention may be used in the form of a suitable combined body. When the reverse hemagglutination (R-PHA) method is used, for example, the antibody of the present invention is combined with fine carrier particles. The erythrocytes of mammals or birds are preferred as the fine carrier particles but others such as polystyrene latex, polyester latex, vinyl chloride, bentonite, glass beads, may also be used. The antibody of the present invention can be combined with these fine carrier particles by use of glutaraldehyde, formaldehyde, tannic acid, bisdiazotized benzidine, chromium chloride or carbodiimide.

When the radioimmunoassay (RIA) method is used, the antibody of the present invention must be labelled with an isotope. $^{125}$I and $^{131}$I can be used as the isotope and can be combined with the antibody of the present invention by the chloramine T method.

When the enzyme-linked, immunoadsorbent (ELISA) method is used, the antibody of the present invention must be combined with an enzyme. Examples of such enzymes are glucose oxidase, alkali phosphatase, peroxidase or β-galactosidase. Glutaraldehyde can be used as the coupling agent for this purpose.

The diagnostic reagent of the present invention can be used not only for the diagnosis of non-A, non-B hepatitis but also as an experimental detection or determination reagent for non-A, non-B hepatitis-associated antigen as well as for one-A, non-B hepatitis-associated antibody. For this reason, the term "diagnostic reagent" in the present invention should not be interpreted too narrowly but be interpreted to embrace these reagents.

The following experimental examples will illustrate the usefulness of the monoclonal antibody and reagent of the present invention.

Experimental Example 1
Samples:
(a) Non-A, non-B hepatitis-associated antigen obtained by isolation and purification from non-A, non-B hepatitis autopsy liver and having the following physicochemical properties (hereainafter referred to as "AN-6520"):
molecular weight: 1,500,000 or more (gel filtration) sedimentation constant $(10^{-13})$: 51.5S (ultracentrifugal analysis)
buoyant density $(g/cm^3)$: 1.15—1.25 (in cesium chloride and in potassium bromide)
particle diameter (nm): 26—37
electrophoretic mobility: $\alpha_2$—$\alpha_1$ globulin region (in agarose gel).
(b) serum of non-A, non-B hepatitis patients (antibody positive against AN-6520)
(c) purified antibody from the serum of the immunized rabbit described in the item "Preparation of Purified Antibody" in Example 1-(1); hereinafter referred to as "AN-6520 antibody".

**0 092 249**

(d) ascitic serum obtained in Example 2 and produced from the hybridoma AT-F-1 obtained in Example 1.

(e) ascitic serum obtained in Example 2 and produced from the hybridoma AT-F-3 obtained in Example 1.

Method:

0.8% (w/v) of Agarose® A-45 (Nakarai Kagaku), 0.02M EDTA · 3Na, 0.1M sodium chloride and 0.1% $NaN_3$ were dissolved in a Tris-HCl buffer (0.01M, pH 7.5) to prepare a 1.5 mm-thick agarose gel plate. A detection test was carried out for each sample (a) through (e) in accordance with the micro-Ouchterlony's method.

Results:

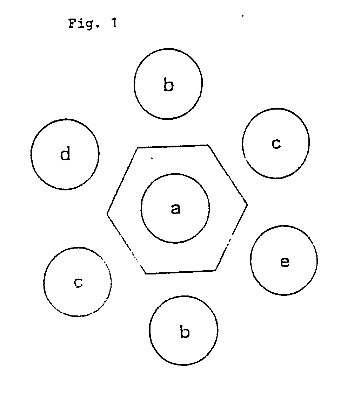

The results are shown in Figure 1, which is a schematic view showing the state of occurrence of precipitin lines generated by the reaction inside the agarose gel. Round frames *a* through *e* represent arrangements of areas in which the corresponding samples are spotted. It can be observed from Figure 1 that the monoclonal antibody of the present invention in the ascitic serum forms clear precipitin lines with the antigen AN-6520 and that each of the precipitin lines merges with those formed between AN-6520 and the AN-6020 antibodies from the serum of non-A, non-B hepatitis patients and from that of the immunized rabbit. It can be thus understood that the monoclonal antibody of the present invention exbibits a specific antigen-antibody reaction with the non-A, non-B hepatitis-associated antigen and this specificity is peculiar to other non-A, non-B hepatitis-associated antibodies and has immunological identity.

Experimental Example 2

Roughened surface glass beads (diameter 5.0±0.2 mm) prepared in the manner which will be described in Example 2 and sensitized by the monoclonal antibody of the present invention were placed in a test tube and 100 µl each of AN-6520 solutions having varying concentrations were added. The mixtures were left standing at 40°C for 2 hours so as to sufficiently couple the antibody with the glass beads. The uncoupled protein was then washed three times with a 0.1% PBS solution of Tween 20. The monoclonal antibody, prepared in advance in the manner which will be described in Example 5 and labelled with [125]I, was diluted with 1% BSA (containing 0.1% $NaN_3$) to give a count of $1 \times 10^5$ cpm. 100 µl of this solution was added to the glass beads described above, and was left standing at 40°C for 1 hour. After washed three times with the 1% PBS solution of Tween 20, each sample was counted.

Results:

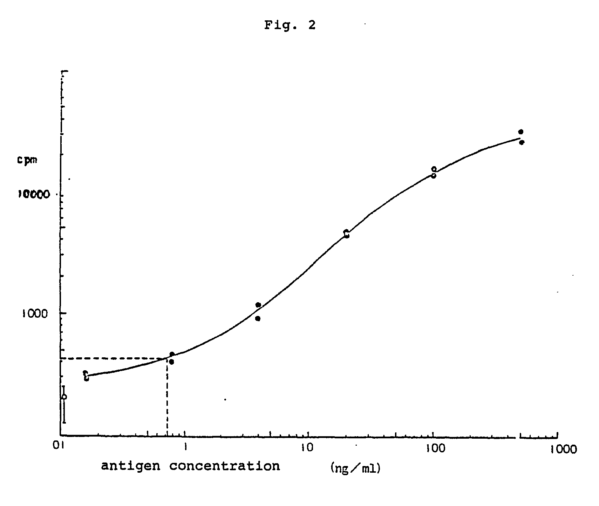

The results are shown in Figure 2. The count was 200 cpm on the average when the concentration of AN-6520 was 0 ng/ml (control). The measured sensitivity of AN-6520 in the test method described above was found to be 0.75 ng/ml because the possiblity of being antigen-positive was generally recognized when an S/N ratio was at least 2.1 (where S represents a cpm value of each sample and N does that of the control). In other words, the monoclonal antibody of the present invention makes it possible to carry out the test having a sensitivity of as high as 0.75 ng/ml.

Experimental Example 3
Samples:

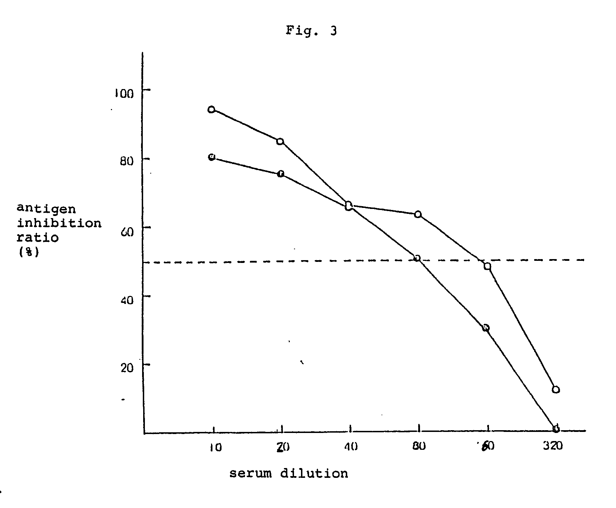

Serums A and B collected from two non-A, non-B hepatitis patients were diluted in the multiples of dilution plotted on the abscissa of Figure 3 to use as the samples. Serum N (number of samples: 4) that was antibody-negative and serum P (number of samples: 4) that showed an antibody titer of at least 32 times as high, when measured in advance by the PHA method, were used as the controls.

Method:

A serum dilution was prepared by adding 40 µl of an RIA buffer (prepared by dissolving 1% BSA and 0.1% $NaN_3$ in PBS) to 10 µl of each sample. Separately, an antigen solution was prepared by dissolving AN-6520 in the RIA buffer in 200 ng/ml. 50 µl of each serum dilution and 50 µl of the antigen solution were placed in a test tube and left standing at 37°C for 60 minutes and at 4°C for day and night. Glass beads that were prepared in accordance with the method of Example 4 were added to each test tube and left standing at 40°C for 2 hours. The content was washed three times with a 0.1% PBS solution of Tween 20. The solution was adjusted to a count of $1 \times 10^5$ cpm in the manner described in Experimental Example 2. After 10 µl of an [125]I-labelled antibody solution was added, the mixture was left standing at 40°C for one hour and then washed three times with a 1% PBS solution of Tween 20. Each sample was thereafter counted. The antigen inhibition ratio was calculated for each sample in accordance with the following formula:

$$\text{antigen inhibition ratio} = 100 \times \frac{\bar{N}\text{cpm} - \text{Scpm}}{\bar{N}\text{cpm} - \bar{P}\text{cpm}}$$

6

where Scpm is a count for the sample, and $\bar{N}cpm$ and $\bar{P}cpm$ are mean values of counts for the serums N and P, respectively.

Separately, antibody titers of the serums A and B were measured by the PHA method.

Results:

The results are shown in Figure 3, which illustrates the relation between the multiples of dilution of the sample serum and the antigen inhibition ratio. Line marked by O refers to the serum A and line marked by O refers to the serum B. The antibody titer was 16 times for the serum A and 8 times for the serum B when measured by the PHA method.

Generally, a serum is recognized as antibody-positive when the antigen inhibition ratio exceeds 50%; hence, the serum A was recognized as antibody-positive up to a dilution of 160 times and the serum B up to a dilution of 80 times when measured by the measuring method described above. When measured by the PHA method, on the other hand, the limit was found to be 16 times for the serum A and 8 times for the serum B. Accordingly, the sensitivity was improved by about 10 times by the measuring method described above. It can be thus appreciated that the diagnostic reagent containing the monoclonal antibody of the present invention as its principal ingredient and utilizing the RIA method as the measuring method had high usefulness.

Experimental Example 4

The antigen inhibition ratios were determined for serums of various hepatitis patients in accordnce with the method described in the item "Method" in Experimental Example 3 and when the antigen inhibition ratios exceeded 70%, there were recognized as being antibody-positive. The results are illustrated in Table 1 for each pathogenic virus and disease.

TABLE 1

| Disease \ Pathogenic virus | Non-A, non-B type virus | Type B virus | Type A virus | Viruses other than those described and non-virus |
|---|---|---|---|---|
| acute hepatitis | 4/9 (44.4) | 0/6 | 0/3 | 0/3 |
| chronic hepatitis | 5/16 (31.3) | 0/11 | | 0/2 |
| hepato-cirrhosis | 0/3 | 0/5 | | |
| primary hepatoma | 0/2 | | | |
| metastatic hepatoma | | | | 0/1 |
| total | 9/30 (30) | 0/22 | 0/3 | 0/6 |

In this table, figures of the denominator represent the number of cases, those of the numerator represent the number of positive cases and those in parentheses represent the percentage of positive cases. From Table 1, it can be appreciated that the diagnostic reagent of the present invention can specifically detect non-A, non-B hepatitis.

Experimental Example 5
Samples:

Twelve hybridomas (12 kinds of hybridomas described in the column "Hybridoma" of Table 2 producing the monoclonal antibody were selected for twelve clones having an agglutination antibody titer of at least 4 times among the clones of Example 1 and intraperitoneal incubation was carried out in the manner described in Example 2. The resulting ascitic serums were used as the samples. The control was prepared in the following manner. Pulpar cells of a BALB/C mouse immunized to a Friend's leukemia virus and NS-1 were fused and the resulting hybridoma (listed as "A-12" in the column "Hybridoma" in Table 2) was intraperitoneally incubated in the manner described in Example 2 to obtain an ascitic serum (monoclonal antibody $I_gG$).

7

Method:

The following procedures (1) through (3) were carried out for each sample.

(1) The antibody titer was determined by the PHA method described in Example 1.

(2) The formation of precipitin lines was observed for samples of 10-fold and 1000-fold dilutions by the MO method described in the item "Method" in Example 1.

(3) After each sample was diluted 1,000 times, the inhibition ratio for the added antigen was determined by the RIA method described in the item "Method" in Experimental Example 1.

Results:

The results are illustrated in Table 2. In the description of the MO method, symbols have the following meaning:

++: strongly positive
+: positive
±: weakly positive
−: negative.

TABLE 2

| Hybridoma identifi- cation | PHA antibody titer | Precipitin line by the MO method | | Antigen inhibition ratio by RIA method |
|---|---|---|---|---|
| | | dilution 10× | dilution $10^3$× | |
| AT-F-1 | $10^5$ | ++ | − | 89% |
| AT-F-2 | $10^6$ | ++ | ++ | 97% |
| AT-F-3 | $10^6$ | ++ | + | 92% |
| AT-F-4 | $10^6$ | ++ | + | >99.5% |
| AT-F-5 | $10^5$ | ++ | − | 57.5% |
| AT-F-6 | $10^6$—$10^7$ | ++ | + | >99.5% |
| AT-F-7 | $10^6$ | ++ | ± | 98.5% |
| AT-F-9 | $10^5$ | ++ | + | 78.5% |
| AT-F-10 | $10^6$ | ++ | + | >99.5% |
| AT-F-11 | $10^6$ | ++ | − | >99.5% |
| AT-F-12 | $10^6$ | ++ | + | 99.2% |
| AT-F-13 | $10^5$ | ++ | − | 88.5% |
| A-12 | — | — | — | 0% |

It can be appreciated from Table 2 that all of the monoclonal antibodies of the present invention have high titers.

The present invention will be described in more detail with reference to the following examples thereof.

Example 1

AN-6520 was dissolved in a phosphate buffered saline (PBS) to prepare 85 μg/ml of an antigen solution. 0.2 ml of the antigen solution and 0.2 ml of a Freund's complete adjuvant were mixed and subcutaneously dosed to BALB/C mice. 100 days later, 0.2 ml of the antigen solution was intravenously dosed to the same mice. Three days later, the spleen of each mouse was enucleated and was finely milled in an RPMI-1640 medium. The resulting cell suspension was washed three times in RPMI-1640 by centrifugal separation and was again suspended in RPMI-1640. $1.28 \times 10^7$ pulpar cells and $3.2 \times 10^7$ myeloma cells (NS-1) that were washed once in advance in RPMI-1640 were mixed and 800 g of the mixture was centrifuged for 5 minutes to prepare a pellet and to remove the medium. 1 ml of a solution prepared by dissolving Polyethylene glycol-4000 (Nakarai Kagaku) in RPMI-1640 in 50% w/v was added dropwise to the pellet with stirring and 10 ml of RPMI-1640 was gradually added in the course of 5 to 10 minutes while stirring was continued. Finally, 400 g of the mixed solution was centrifuged for 5 minutes to remove polyethylene glycol and was suspended again in 64 ml of the RPMI-1640 medium containing 15% v/v of a fetal calf serum. Next, 0.2 ml (number of cells: $5 \times 10^5$) each of the suspension described above was placed in 315 holes of 96-hole tissue culture plates and the half of the incubation supernatant was replaced by an HAT medium (an RPMI-1640 medium to which 136.1 mg/ml of hypoxanthine, 1.76 mg/ml of aminopterin, 38.75 mg/ml of thymidine and 15% v/v of the fetal calf serum were added) every two to three days from the next day (HAT selection). Ten days later, the presence of fused cells was observed in 150 holes out of 315 holes. The incubation supernatant was screened in accordance with the PHA method and the holes having the agglutination antibody titer of at least 4 times were found to be 16 holes.

Each positive cell of these 16 holes was diluted by the RPMI-1640 medium supplemented with 15% v/v of the fetal calf serum and with $3 \times 10^6$/ml of the thymocites of the BALB/C mouse and 0.2 ml (number of cells: 3) each of the dilution was placed in each hole of a 96-hole tissue culture plate for incubation. The incubation supernatant was screened in accordance with the PHA method as the cloning operation so as to select the antibody-producing cell strain (limiting dilution). This cloning operation was repeated twice for each antibody-positive cell at such a dilution ratio at which one cell was placed in one hole. Finally, antibody-producing fused cell strains of 14 clones could be obtained. The hybridomas for them were named AT-F-1 through AT-F-14, respectively.

20 ml of a saturated ammonium sulfate solution was added to 20 ml of the incubation supernatant of each cell strain and the mixture was left standing at room temperature for one hour and was then centrifuged at 9,000 r.p.m. for 20 minutes. The resulting precipitate was dissolved again in 0.5 ml of PBS and was dialyzed by PBS for one day. The sub-classes of the immunoglobulin were determined for the resulting concentrated protein solution by the MO method and by the gel filtration method. It was found to be $I_gG1$ and $I_gM$. Screening by the PHA method was carried out in the following manner.

(1) Preparation of purified antigen:

About 5 g of the autopsy liver of a non-A, non-B hepatitis patient was sliced and homogenized by adding 20 ml of Tris-HCl buffered saline (0.01M, pH 7.5). Next, the solution was centrifuged at 10,000 r.p.m. for 20 minutes and the homogenate supernatant was collected to obtain a liver homogenate supernatant before isolation and purification.

10 ml of the liver homogenate supernatant was charged in a column (2.6×90 cm) packed with Sephacryl S-200 (a product of Pharmacia) equilibrated in advance with a Tris-HCl buffered saline (0.01M, pH 7.5), and was eluted by a Tris-HCl buffered saline (0.01M, pH 7.5). A fraction corresponding to the first peak having an antigen activity was pooled by continuously monitoring the eluate with ultraviolet absorption at 280 nm. The antigen activity was determined by agar gel diffusion. The fraction thus collected was concentrated by ultrafiltration (PM-10, a product of Amicon Corporation.

The resulting concentrate was subjected to elution in the same manner as described above but using Sephacryl S-300 (a product of Pharmacia) in place of the Sephacryl S-200, and was again concentrated by ultrafiltration.

5 ml of the resulting concentrate was subjected to sucrose density gradient centrifugation (24,000 r.p.m., 16 hours). The density gradient ranged from 5 to 60% (w/v) and 10 ml each at 5%, 15%, 25% and 35% and 5 ml each at 45% and 60% were obtained. The concentrate was added to the uppermost portion. After the treatment, fractionation was carried out at a rate of 2.5 ml/tube and a fraction corresponding to a sucrose density of 25 to 40% and having an antigen activity was pooled. The fraction thus pooled was dialyzed by a Tris-HCl buffered saline (0.01M, pH 7.5) and was concentrated by ultrafiltration (PM-10, a product of Amicon Corporation).

Next, the resulting concentrate was subjected to cesium chloride equilibrated density gradient centrifugation (40,000 rpm, 20 hours). Samples of 4.5 ml each were prepared within the density gradient range of 1.00 to 1.50 g/cm$^3$ and the concentrate was added to the uppermost portion. After the treatment, fractionation was effected at a rate of 0.2 ml/tube from the upper tube layer and a fraction corresponding to cesium chloride density of 1.15 to 1.25 g/cm$^3$ and having an antigen activity was pooled. The specific gravity of each fraction was measured with an Abbe's refractometer and each fraction pooled was dialyzed by a Tris-HCl buffered saline (0.01M, pH 7.5).

The physicochemical properties of the substance contained in the resulting solutions are tabulated below and the substance was used as the purified antigen:

molecular weight: 1,500,000 or more (gel filtration)
sedimentation constant ($10^{-13}$): 51.5S (ultracentrifugal analysis)
buoyant density (g/cm$^3$): 1.15—1.25 (in cesium chloride and in potassium bromide)
particle diameter (nm): 26—37
electrophoretic mobility: $\alpha_2$—$\alpha_1$ globulin region (in agarose gel).

(2) Preparation of sensitized erythrocyte:

Sheep blood was charged in a centrifugal tube and centrifugation was repeated five times at 2,000

9

r.p.m. for 10 minutes using a saline to wash the erythrocytes. A phosphate buffered saline of pH 7.5 and 0.15M was added to the erythrocytes in a 5% concentration. One-fifth by volume of a glutaraldehyde solution prepared in a 2.5% concentration using the same phosphate buffered saline was added to a suspension of the above-mentioned erythrocytes, and the mixture was reacted with stirring at room temperature for about 5 hours to fix the erythrocytes. Next, this solution was centrifuged to obtain fixed erythrocytes, which were washed several times by centrifugation using the saline. The fixed erythrocytes were then prepared in the form of a 5% suspension using the above phosphate buffer and the same quantity of a tannic acid solution prepared in 5 mg/dl using the same phosphate buffered saline was added to the solution. The mixed solution was then stirred for 30 minutes, and centrifuged to obtain tannic acid-treated, fixed erythrocytes, which were further washed several times by centrifugation using a saline. The phosphate buffer was added to the tannic acid-treated, fixed erythrocytes, thereby forming a 5% erythrocyte suspension.

The erythrocyte suspension was mixed with the same quantity of a solution obtained by preparing the solution of the purified antigen in a protein concentration of about 10 µg/ml (antibody dilution ratio 1:20) using the phosphate buffered saline, and was stirred at room temperature for 60 minutes to achieve sensitization. The solution was centrifuged to obtain sensitized blood cells, which were washed several times by centrifugation using the saline. A phosphate buffered saline containing 2% normal rabbit serum was added to the resulting sensitized blood cells to obtain a 7% blood cell suspension. This sensitized blood cell suspension was used as a diagnostic reagent in accordance with the PHA method

(3) Method:

25 µl of a phosphate buffered saline (0.15M, pH 7.5) containing 2% normal rabbit serum was added dropwise into a microtitration plate (V-type) made of acryl resin and the test serum was diluted by $2^n$ times in two series. 25 µl of a phosphate buffered saline (0.15M, pH 7.5) containing 2% normal rabbit serum was added to one of the series while 25 µl of a 100-fold dilution of the purified antigen was added to the other. After incubation at 37°C for 30 minutes, the sensitized blood cells were added and the plate was further incubated at room temperature for 2 hours to confirm the occurrence of agglutination. The anti-body titer was expressed as the reciprocal of the highest dilution of the sample that showed hemagglutination of the purified antigen.

Example 2

0.5 ml of pristane (2,6,10,14-tetramethylpentadecane) was intraperitoneally dosed to a BALB/C mouse and 2 to 30 days later, the antibody-producing strain (corresponding to the cell number of $2$—$4 \times 10^6$) obtained in Example 1 was intraperitoneally dosed to the same mouse. Two to three weeks later, the ascitic serum was collected. When measured by the PHA method, it was found to have an agglutination antibody titer of about $10^5$—$10^7$. The ascitic serum was a nutrient composition containing the monoclonal antibody of the present invention.

Example 3

2 ml of the ascitic serum obtained in Example 2 was charged in a Sephacryl S-200 column ($26 \times 36$ cm) and was fractionated in 5 ml fractions using an eluent prepared by adding 0.015M sodium chloride, 0.002M of EDTA-3Na and 0.1% NaN$_3$ to a 0.01M Tris-HCl buffer (pH 7.5). Each fraction was screened by the PHA method and fractions that were antibody positive were pooled to obtain the purified matter. The yield was 34.4 mg as IgG. The product was adjusted in a protein content of about 1 mg/ml and was freeze-preserved. When measurement by the PHA method at this concentration was carried out, the agglutination antibody titer was found to be 60,000 times.

Example 4

Roughened surface glass beads (diameter: about 5 mm) were placed in a 2% acetone solution of 3-aminopropyltriethoxysilane, stirred at room temperature for 30 minutes and washed by PBS. Next, a 10% PBS solution of glutaraldehyde was added and the mixture was stirred at room temperature for 3 hours and then washed by PBS. 12.5 µg/ml of the purified monoclonal antibody obtained in Example 3 was added to the resulting aldehyde glass beads, left standing at 4°C for a night, again washed with PBS and thereafter stored in a 5% BSA solution (containing 0.1% NaN$_3$) at 4°C.

The product obtained in this Example was glass beads that were surface-coated with the monoclonal antibody and could be used as a diagnostic reagent utilizing the antibody sandwich method.

Example 5

$^{125}$I-Na of 0.5 mCi ($1.85 \cdot 10^7$ S$^{-1}$) and 0.29 ml of a 0.05M phosphate buffered saline (pH 7.5) were added to 10 µl of the purified monoclonal antibody (protein content of 1 mg/ml) obtained in Example 3, and 2 µl of a solution obtained by dissolving chloramine T in distilled water at a rate of 2.5 mg/ml was further added. The mixture was stirred at 4°C for 5 minutes. Next, a solution prepared by dissolving sodium metabisulfite in distilled water at a rate of 2.5 mg/ml was added to stop the reaction. An $^{125}$I-labelled substance was immediately isolated from free $^{125}$I by centrifugal gel filtration using Biogel® P-10. The specific activity of

the resulting $^{125}$I-labelled substance was found to be approximately 10 to 20 µCi/µg (3.7—7.4 · 10 S$^{-1}$/µg). This $^{125}$I-labelled substance was used as a diagnostic reagent utilizing the RIA method.

Example 6

The purified monoclonal antibody obtained in Example 3 was concentrated in 5—10 mg/ml and 0.3 ml of alkali phosphates (5 mg/ml) was added to and stirred with 0.1 to 0.2 ml of the concentrate. Next, 50 µl of a 2.5% glutaraldehyde solution was added, followed by stirring at room temperature for 30 minutes. The solution was dialyzed by a Tris-HCl buffer (pH 8.0) for a day and night, and centrifuged at 2,000 r.p.m. for 10 minutes. The supernatant was used as an enzyme-linked antibody for a diagnostic reagent utilizing the EIA method.

Example 7

Sheep blood was charged in a centrifugal tube and centrifugation was repeated five times at 2,000 r.p.m. for 10 minutes using a saline to wash the erythrocytes. A phosphate buffered saline of pH 7.5 and 0.15M was added to the erythrocytes in a 5% concentration. One-fifth by volume of a glutaraldehyde solution prepared in a 2.5% concentration using the same phosphate buffered saline was added to a suspension of the above-mentioned erythrocytes, and the mixture was reacted under stirring at room temperature for about 5 hours to fix the erythrocytes. Next, this solution was centrifuged to obtain fixed erythrocytes, which were washed several times by centrifugation using the saline. These fixed erythrocytes were then prepared in the form of a 5% suspension using the above-mentioned phosphate buffer and the same quantity of a tannic acid solution prepared in 5 mg/ml using the same phosphate buffered saline was added to the solution. The mixed solution was then stirred for 30 minutes, and centrifuged to obtain tannic acid-treated, fixed erythrocytes, which were further washed several times by centrifugation using the saline. The phosphate buffer was added to the tannic acid-treated, fixed erythrocytes, thereby forming a 5% erythrocyte suspension.

The erythrocyte suspension was mixed with the same quantity of a solution obtained by diluting the purified monoclonal antibody obtained in Example 3 in a protein concentration of about 10 µg/ml using the phosphate buffered saline, and was stirred at room temperature for 60 minutes to achieve sensitization. The solution was centrifuged to obtain sensitized blood cells, which were then washed several times by · centrifugation using the saline. A phosphate buffered saline containing 2% normal rabbit serum was added to the resulting sensitized blood cells to obtain a 7% blood cell suspension. This sensitized blood cell suspension was used as a diagnostic reagent utilizing the R-PHA method.

**Claims**

1. A non-A, non-B hepatitis-associated monoclonal antibody exhibiting a specific antigen-antibody reaction with a non-A, non-B hepatitis-associated antigen obtained by isolation and purification from non-A, non-B hepatitis autopsy liver, said antigen having the following physicochemical properties:
   molecular weight: 1,500,000 or more (gel filtration),
   sedimentation constant ($10^{-13}$): 51.5S (ultracentrifugal analysis),
   buoyant density (g/cm$^3$): 1.15~1.25 (in cesium chloride and in potassium bromide)
   particle diameter (nm): 26~37
   electrophoretic mobility: $\alpha_2$—$\alpha_1$ globulin region (in agarose gel),
   said antigen having the following immunological properties: it undergoes a precipitation reaction in an agarose gel with the serum of covalescent non-A, non-B hepatitis patients, sheep erythrocytes sensitized by said antigen exhibit specific and highly sensitive passive hemaglutination with the serum of covalescent non-A, non-B hepatitis patients, it does not react with HAV antibody, anti-HBs, anti-HBc, anti-HBe, anti-δ, HC antibody, HB antibody, Arai antibody, F antibody, normal human liver supernatant antibody and normal human plasma antibodies.

2. The non-A, non-B hepatitis-associated monoclonal antibody as defined in Claim 1 wherein said monoclonal antibody is produced by in vivo or in vitro incubation of a hybridoma, said hybridoma being obtained by the steps of sensitizing an animal by said non-A, non-B hepatitis-associated antigen of Claim 1, collecting an antibody-producing cell from said animal, fusing said cell with a myeloma cell, selecting in an HAT medium, screening as regards the antibody producibility and then carrying out cloning.

3. The non-A, non-B hepatitis-associated monoclonal antibody as defined in Claim 1 or 2 wherein said animal is a mouse.

4. The non-A, non-B hepatitis-associated monoclonal antibody as defined in any of Claims 1 through 3 wherein said antibody-producing cell is a pulpar cell.

5. The non-A, non-B hepatitis-associated monoclonal antibody as defined in any of Claims 1 through 4 wherein said meyloma cell is a murine cell.

6. The non-A, non-B hepatitis-associated monoclonal antibody as defined in any of Claims 1 through 5 wherein said cell fusion is effected using polyethylene glycol as a fusing agent.

7. The non-A, non-B hepatitis-associated monoclonal antibody as defined in any of Claims 1 through 6 wherein said cloning is effected by limiting dilution.

8. The non-A, non-B hepatitis-associated monoclonal antibody as defined in any of Claims 2 through 7 wherein said non-A, non-B hepatitis-associated monoclonal antibody is provided in the form of an incubation supernatant of said hybridoma defined in any of Claims 2 through 7.

9. The non-A, non-B hepatitis-associated monoclonal antibody as defined in any of Claims 2 through 7 wherein said non-A, non-B hepatitis-associated monoclonal antibody is provided in the form of an ascitic serum obtained by dosing an animal having a suitable tissue with said hybridoma defined in any of Claims 2 through 7.

10. A diagnostic reagent for non-A, non-B hepatitis, containing, as its essential ingredient, a non-A, non-B hepatitis-associated monoclonal antibody exhibiting a specific antigen-antibody reaction with a non-A, non-B hepatitis-associated antigen obtained by isolation and purification from non-A, non-B hepatitis autopsy liver and having the following physicochemical properties:

molecular weight: 1,500,000 or more (gel filtration)
sedimentation constant $(10^{-13})$: 51.5S (ultracentrifugal analysis)
buoyant density $(g/cm^3)$: 1.15~1.25 (in cesium chloride and in potassium bromide)
particle diameter (nm): 26—37
electrophoretic mobility: $\alpha_2$—$\alpha_1$ globulin region (in agarose gel).

11. The diagnostic reagent for non-A, non-B hepatitis as defined in Claim 10 wherein said diagnostic reagent utilizes the reverse passive hemagglutination method.

12. The diagnostic reagent for non-A, non-B hepatitis as defined in Claim 11 wherein said reverse passive hemagglutination method uses sheep sensitized erythrocytes.

13. The diagnostic reagent for non-A, non-B hepatitis as defined in Claim 10 wherein said diagnostic reagent uses the antibody sandwich method.

14. The diagnostic reagent for non-A, non-B hepatitis as defined in Claim 13 wherein said antibody sandwich method uses sensitized glass beads and an $^{125}$I-labelled antibody.

15. The diagnostic reagent for non-A, non-B hepatitis as defined in Claim 13 wherein said antibody sandwich method uses a sensitized solid phase and an alkaliphosphatase-labelled antibody.


**Patentansprüche**

1. Monoklonaler Antikörper auf nicht-A, nicht-B-Hepatitis, welcher eine spezifische Antigen-Antikörper-Reaktion mit einem nicht-A, nicht-B-Hepatitis-assoziierten Antigen eingeht, wobei letzteres erhalten wurde durch Isolierung und Reinigung aus nicht-A, nicht-B-Hepatitis-Autopsieleber, und das genannte Antigen die folgenden physiko-chemischen Eigenschaften aufweist:

Molekulargewicht: 1.500.000 oder darüber (Gelfiltration),
Sedimentationskonstante $(10^{-13})$: 51,5S (analytische Ultrazentrifugation),
Auftriebsdichte $(g/cm^3)$: 1,15~1,25 (in Cäsiumchlorid und in Kaliumbromid),
Teilchendurchmesser (nm): 26~37,
elektrophoretische Beweglichkeit: $\alpha_2$—$\alpha_1$-Globulinbereich (in Agarosegel),

wobei das Antigen die folgenden immunologischen Eigenschaften aufweist: es unterliegt einer Präzipitationsreaktion in einem Agarosegel mit dem Serum kovaleszenter nicht-A, nicht-B-Hepatitis-Patienten; Schaf-Erythrozyten, die mit dem genannten Antigen sensibilisiert wurden, zeigen spezifische und hoch empfindliche passive Hämagglutination mit dem Serum kovaleszenter nicht-A, nicht-B-Hepatitis-Patienten; es reagiert nicht mit HAV-Antikörper, anti-HBs, anti-HBc, anti-HBe, anti-δ, HC-Antikörper, HB-Antikörper, Arai-Antikörper, F-Antikörper, Antikörper im Überstand von normaler menschlicher Leber und Antikörper von normalem menschlichen Plasma.

2. Monoklonaler Antikörper auf nicht-A, nicht-B-Hepatitis nach Anspruch 1, wobei der genannte monoklonale Antikörper durch in vivo oder in vitro Inkubation eines Hybridoms hergestellt wird, und das genannte Hybridom mit Hilfe folgender Schritte erhalten wird:

Sensibilisieren eines Tieres mit dem genannten nicht-A, nicht-B-Hepatitis-assoziierten Antigen nach Anspruch 1, Gewinnung einer Antikörper-produzierenden Zelle aus dem genannten Tier, Fusion dieser Zelle mit einer Myelomzelle, Selektion in einem HAT-Medium, Screenen in bezug auf die Fähigkeit zur Antikörperbildung, und Durchführen einer Clonierung.

3. Monoklonaler Antikörper auf nicht-A, nicht-B-Hepatitis, wie er in Anspruch 1 oder 2 definiert ist, wobei das Tier eine Maus darstellt.

4. Monoklonaler Antikörper auf nicht-A, nicht-B-Hepatitis, wie er in einem der Ansprüche 1 bis 3 definiert ist, wobei die genannte Antikörper-produzierende Zelle eine Markzelle darstellt.

5. Monoklonaler Antikörper auf nicht-A, nicht-B-Hepatitis, wie er in einem oder mehreren der Ansprüche 1 bis 4 definiert ist, wobei die genannte Myelomzelle eine Zelle der Maus darstellt.

6. Monoklonaler Antikörper auf nicht-A, nicht-B-Hepatitis, wie er in einem oder mehreren der Ansprüche 1 bis 5 definiert ist, wobei die Zellfusion unter Verwendung von Polyethylenglykol als Fusionierungsagens durchgeführt wird.

7. Monoklonaler Antikörper auf nicht-A, nicht-B-Hepatitis, wie er in einem oder mehreren der Ansprüche 1 bis 6 definiert ist, wobei die genannte Clonierung durch limitierende Verdünnung erfolgt.

8. Monoklonaler Antikörper auf nicht-A, nicht-B-Hepatitis, wie er in einem oder mehreren der Ansprüche 2 bis 7 definiert ist, wobei der genannte nicht-A, nicht-B-Hepatitis-assoziierte monoklonale Antikörper in Form eines Inkubationsüberstandes des genannten Hybridoms, wie es in einem oder mehreren der Ansprüche 2 bis 7 definiert ist, zur Verfügung gestellt wird.

9. Monoklonaler Antikörper auf nicht-A, nicht-B-Hepatitis, wie er in einem oder mehreren der Ansprüche 2 bis 7 definiert ist, wobei der genannte nicht-A, nicht-B-Hepatitis-assoziierte monoklonale Antikörper in Form von Ascites-Serum, das erhalten wird durch Verabreichen des Hybridoms, wie es in einem oder mehreren der Ansprüche 2 bis 7 definiert ist, an ein Versuchstier mit einem geeigneten Gewebe, zur Verfügung gestellt wird.

10. Diagnostisches Reagens auf nicht-A, nicht-B-Hepatitis, welches als wesentlichen Bestandteil einen nicht-A, nicht-B-Hepatitis-assoziierten monoklonalen Antikörper enthält, der eine spezifische Antigen-Antikörper-Reaktion mit einem nicht-A, nicht-B-Hepatitis-assoziierten Antigen zeigt, wobei letzteres erhalten wird durch Isolierung und Reinigung aus nicht-A, nicht-B-Hepatitis-Autopsieleber, und die folgenden physiko-chemischen Eigenschaften aufweist:

Molekulargewicht: 1.500.000 oder darüber (Gelfiltration)
Sedimentationskonstante ($10^{-13}$): 51.5S (analytische Ultrazentrifugation),
Auftriebsdichte (g/cm$^3$): 1,15~1,20 (in Cäsiumchlorid und in Kaliumbromid),
Teilchendurchmesser (nm): 26~37,
elektrophoretische Beweglichkeit: $\alpha_2$—$\alpha_1$-Globulinbereich (in Agarosegel).

11. Diagnostisches Reagens auf nicht-A, nicht-B-Hepatitis nach Anspruch 10, wobei das genannte diagnostische Reagens von der reversen passiven Hämagglutinations-Methode Gebrauch macht.

12. Diagnostisches Reagens auf nicht-A, nicht-B-Hepatitis nach Anspruch 11, wobei zur reversen passiven Hämagglutinations-Methode sensibilisierte Erythrozyten von Schaf verwendet werden.

13. Diagnostisches Reagens auf nicht-A, nicht-B-Hepatitis nach Anspruch 10, wobei das genannte diagnostische Reagens von der Antikörper-Sandwich-Methode Gebrauch macht.

14. Diagnostisches Reagens auf nicht-A, nicht-B-Hepatitis nach Anspruch 13, wobei zur Antikörper-Sandwich-Methode sensibilisierte Glaskügelchen und ein $^{125}$I-markierter Antikörper verwendet werden.

15. Diagnostisches Reagens auf nicht-A, nicht-B-Hepatitis nach Anspruch 13, wobei zur Antikörper-Sandwich-Methode eine sensibilisierte Festphase und eine mit Alkaliphosphatase markierter Antikörper verwendet werden.

**Revendications**

1. Anticorps monoclonal associé à l'hépatite non-A, non-B donnant lieu à une réaction antigène-anticorps spécifique avec un antigène associé à l'hépatite non-A, non-B, obtenu par isolement et purification à partir d'un foie atteint d'hépatite non-A, non-B, prélevé par autopsie, ledit antigène ayant les propriétés physico-chimiques suivantes:

Poids moléculaire: 1 500 000 ou plus (filtration sur gel)
Constante de sédimentation ($10^{-13}$): 51,5S (analyse par ultra-centrifugation)
Masse volumique apparente (g/cm$^3$): de 1,15 à 1,25 (dans du chlorure de césium et dans du bromure de potassium).
Diamètre de particule (nm): de 26 à 37
Mobilité électrophorétique: région des globulines $\alpha_2$—$\alpha_1$ (dans du gel d'agarose),
ledit antigène ayant les propriétés immunologiques suivantes:
il subit une réaction de précipitation dans un gel d'agarose avec le sérum de patients atteints d'hépatite non-A, non-B en phase de convalescence, des éthythrocytes de mouton sensibilisés par l'antigène ont une sensibilité spécifique et élevée à l'hémagglutination passive avec le sérum de patients atteints d'hépatite non-A, non-B en phase de convalescence, il ne réagit pas avec un anticorps dirigé contre le virus de l'hépatite A, un anticorps anti-HBs, anti-HBc, anti-HBe, anti-δ, un anticorps anti-HC, un anticorps anti-HB, un anticorps de Arai, un anticorps antifacteur F, un anticorps de surnageant de foie humain normal ainsi que divers anticorps de plasma humain normal.

2. Anticorps monoclonal associé à l'hépatite non-A, non-B, selon la revendication 1, préparé par incubation in vivo ou in vitro d'un hybridome, ledit hybridome étant obtenu en sensibilisant un animal avec ledit antigène associé à l'hépatite non-A, non-B de la revendication 1, en recueillant une cellule productrice d'anticorps à partir dudit animal, en fusionnant ledit cellule avec une cellule de myélome, en choisissant dans un milieu HAT, en sélectionnant en fonction de la capacité de production d'anticorps et ensuite en effectuant un clonage.

3. Anticorps monoclonal associé à l'hépatite non-A, non-B, selon la revendication 1 ou 2, ledit animal étant une souris.

4. Anticorps monoclonal associé à l'hépatite non-A, non-B, selon l'une quelconque des revendications 1 à 3, ladite cellule productrice d'anticorps étant une cellule de pulpe.

5. Anticorps monoclonal associé à l'hépatite non-A, non-B, selon l'une quelconque des revendications 1 à 4, ladite cellule de myélome étant une cellule murine.

6. Anticorps monoclonal associé à l'hépatite non-A, non-B, selon l'une quelconque des revendications 1 à 5, ladite fusion cellulaire étant effectuée en utilisant du polyéthylèneglycol comme agent de fusion.

7. Anticorps monoclonal associé à l'hépatite non-A, non-B, selon l'une quelconque des revendications 1 à 6, ledit clonage étant effectué par dilution limite.

8. Anticorps monoclonal associé à l'hépatite non-A, non-B, selon l'une quelconque des revendications 2 à 7, caractérisé en ce qu'il est fourni sous la forme d'un surnageant d'incubation dudit hybridome défini à l'une quelconque des revendications 2 à 7.

9. Anticorps monoclonal associé à l'hépatite non-A, non-B, selon l'une quelconque des revendications 2 à 7, caractérisé en ce qu'il est fourni sous la forme d'un sérum ascitique obtenu en administrant à un animal ayant un tissu approprié, une dose dudit hybridome défini à l'une quelconque des revendications 2 à 7.

10. Réactif de diagnostic pour l'hépatite non-A, non-B, contenant comme ingrédient principal, un anticorps monoclonal anti-hépatite non-A, non-B, donnant lieu à une réaction antigène-anticorps spécifique avec un antigène associé à l'hépatite non-A, non-B obtenu par isolement et purification à partir d'un foie atteint d'hépatite non-A, non-B prélevé par autopsie et ayant les propriétés physico-chimiques suivantes:

Poids moléculaire: 1 500 000 ou plus (filtration sur gel)

Constante de sédimentation ($10^{-13}$): 51,5S (analyse par ultra-centrifugation)

Masse volumique apparente ($g/cm^3$): de 1,15 à 1,25 (dans du chlorure de césium et dans du bromure de potassium).

Diamètre de particule (nm): de 26 à 37

Mobilité électrophorétique: région des globulines $\alpha_2$—$\alpha_1$ (dans du gel d'agarose).

11. Réactif de diagnostic pour l'hépatite non-A, non-B selon la revendication 10, ledit réactif de diagnostic utilisant le procédé d'hémagglutination passive inverse.

12. Réactif de diagnostic pour l'hépatite non-A, non-B selon la revendication 11, ledit procédé d'hémagglutination passive inverse utilisant des érythrocytes de mouton sensibilisés.

13. Réactif de diagnostic pour l'hépatite non-A, non-B selon la revendication 10, ledit réactif de diagnostic utilisant le procédé d'essai par anticorps en sandwich.

14. Réactif de diagnostic pour l'hépatite non-A, non-B selon la revendication 13, ledit procédé d'essai par anticorps en sandwich utilisant des perles de verre sensibilisées ainsi qu'un anticorps marque avec $^{125}$I.

15. Réactif de diagnostic pour l'hépatite non-A, non-B selon la revendication 13, le procédé d'essai par anticorps en sandwich utilisant une phase solide sensibilisée et un anticorps marqué par une phosphatase alcaline.

Fig. 1

Fig. 2

Fig. 3